# EUROPÄISCHE PATENTSCHRIFT

(11) **EP 2 124 031 B1**
(45) Veröffentlichungstag und Bekanntmachung des Hinweises auf die Patenterteilung: **01.05.2013**
(21) Anmeldenummer: 09006689.5
(22) Anmeldetag: 19.05.2009
(51) Int. Cl.: G01L 19/00

(54) **Vorrichtung zum Messen des Drucks**
Device for measuring pressure
Dispositif de mesure de pression

(30) Priorität: 20.05.2008 DE 102008024396
(43) Veröffentlichungstag der Anmeldung: 25.11.2009
(73) Patentinhaber: Storz Endoskop Produktions GmbH, 78532 Tuttlingen (DE)
(72) Erfinder: Henes, Rudolf, CH-8200 Schaffhausen (CH); Pauli, Walter, D-78176 Blumberg (DE); Bayer, Andreas, D-78244 Gottmadingen (DE); Krattiger, Beat, CH-8222 Beringen (CH)
(74) Vertreter: Fugmann, Winfried

(56) Entgegenhaltungen:
- EP-A1- 1 813 300
- DE-A1- 4 219 889
- DE-A1- 10 129 455
- US-B2- 6 880 404

## Beschreibung

Die Erfindung betrifft eine Vorrichtung zum Messen des Drucks in einer Fluidleitung mit einer Fluidkammer, die fluidleitend mit der Fluidleitung verbunden ist, und mit einem Druckaufnehmer.

Vorrichtungen der genannten Art sind beispielsweise aus der DE 42 19 889 A1, EP 1 813 300 A1 und der US 6,880,404 B2 bekannt.

Solche Vorrichtungen ermöglichen die Messung des Drucks in Fluidleitugen, insbesondere in Kunststoffschläuchen, die in der Medizintechnik in extrakorporalen Schlauchsystemen bzw. Schlauchbestecken zur Förderung bzw. zum Transport von Fluiden verwendet werden.

Bei den Fluiden handelt es sich beispielsweise um medizinische Spülflüssigkeiten wie Kochsalzlösungen, die zur Reinigung des Innenraums einer Körperhöhle oder eines Gelenks verwendet werden. Es kommen aber auch Gase oder Gasgemische als Fluide in Betracht, die beispielsweise im Rahmen einer laparoskopischen Operation zur Insufflation der Bauchhöhle Verwendung finden.

Das Schlauchsystem bzw. Schlaubesteck wird mit einem medizinischen Gerät, beispielsweise einer Rollenpumpe oder einem Insufflator, zur Förderung des Fluids verbunden.

Ein Schlauchbesteck kann folgende Komponenten umfassen:
- Einen Anschluss zu einem Vorratsbehälter, in dem das zu fördernde Fluid aufbewahrt wird, beispielsweise einen Beutel für Spülflüssigkeit.
- Ein Schlauchsegment, das aus beständigerem Kunststoff bestehen kann, das in das medizinische Gerät eingelegt wird.
- Weitere Kunststoffschläuche, die beispielweise zur Zuleitung bzw. Ableitung, des Fluids verwendet werden.
- Verbindungselemente (Konnektoren) für das Schlauchsegment und die Zu- und Ableitungen des Fluids.
- Einen Anschluss an einen Trokar oder ein Endoskop.

Teile des Schlauchbestecks oder das ganze Schlauchbesteck können als Einmalartikel oder wiederverwendbar ausgeführt sein.

Das Schlauchbesteck muss steril sein, da es mit einem Instrument verbunden wird, dessen distales Ende in die Körperhöhle eines Patienten eingesetzt wird. Daher dürfen Messvornchtungen, die an das Schlauchbesteck angeschlossen werden, die Sterilität des Innenraums des Schlauchbestecks nicht beeinträchtigen. Diese Forderung kann erfüllt werden, in dem die komplette Messvorrichtung steril ausgeführt wird. Dies beinhaltet jedoch das Problem, die gesamte Signalübertragungsstrecke vom Innenraum des Schlauchbestecks bis zur Erfassungseinheit steril ausgestalten zu müssen. Darüber hinaus muss die Erfassungseinheit selbst, beispielsweise ein Druckaufnehmer, steril ausgeführt sein. Dies führt in nachteiliger Weise zu erhöhten Herstellungs- und Betriebskosten der Messvorrichtung.

Alternativ beinhaltet die Signalübertragungsstrecke eine Schnittstelle zwischen einem steril ausgeführten Teil und einem Teil, der nicht steril ausgeführt sein muss. Der steril ausgeführte Teil der Signalübertragungsstrecke nimmt das zu messende Signal, beispielsweise den Fluiddruck, im Innenraum des Schlauchbestecks auf und leitet dies zur Schnittstelle weiter. Durch die Schnittstelle wird der Innenraum des Schlauchbestecks von der Außenwelt steril abgetrennt. Die Erfassungseinheit und der Teil der Signalübertragungsstrecke zwischen Schnittstelle und der Erfassungseinheit können unsteril ausgeführt sein. Dadurch können erhebliche Einsparungen an Kosten und aufwändigen Maßnahmen erzielt werden, da beispielsweise ein Halbleiterdrucksensor nicht autoklavierbar ausgeführt und verbaut werden muss. Die Problemstellung bei diesem alternativen Ansatz besteht darin, eine geeignete Schnittstelle bereitzustellen, die eine gute Signalübertragung gewährleistet und den Innenraum des Schlauchbestecks steril abschirmt. Die Schnittstelle muss eine im Wesentlichen undurchdringliche Barriere für pathogene Keime bilden.

Aus den eingangs zitierten Schriften US 6,880,404 B2, DE 42 19 889 A1 und EP 1 813 300 A1 sind Vorrichtungen bekannt, die eine Fluidkammer aufweisen. Die Fluidkammer ist als Durchlasskammer ausgebildet, die jeweils am Fluideintrittsende und am Fluidaustrittsende mit einer Fluidleitung, die Teil eines Schlauchbestecks sein kann, verbunden ist. Die Fluidkammer ist mit einer Wand versehen, die als flexible Membran ausgebildet bzw. aus elastischem Material geformt ist.

Nachteilig an diesen Ausführungsformen ist, dass die Fluidkammer nicht aus einem Material oder in einem Herstellungsschritt, was beispielsweise bei einem Spritzgussteil aus Kunststoff möglich ist, hergestellt werden kann.

In der Vorrichtung der US 6,880,404 B2 ist bündig an der Wand der Fluidkammer eine weitere Membran angelegt, die Teil eines Gehäuses ist, das einen Druckaufnehmer umfasst. Dadurch wird zwar die Messung sowohl von positiven Drücken (Überdruck gegenüber Umgebungsdruck) als auch negativen Drücken (Unterdruck gegenüber Umgebungsdruck) prinzipiell ermöglicht.

Nachteilig an dieser Ausführungsform ist jedoch, dass kein hydraulisch rigider Kraftschluss zwischen der elastischen Wand der Fluidkammer und der Membran des Druckaufnehmergehäuses vom Druckaufnehmer gegeben ist. Der erfasste Druck, der in Bereichen in der Nähe des Umgebungsdrucks liegt, hängt nicht linear vom Druck ab, mit dem die Fluidleitung beaufschlagt ist. Dadurch sind präzise Messungen von negativen bzw. positiven Drücken in Bereichen des Umgebungsdruckes unmöglich.

In den Vorrichtungen der DE 42 19 889 A1 und EP 1 813 300 A1 ist die Wand durch einen Zwischenraum getrennt gegenüber einem Druckaufnehmer angeordnet. Der Zwischenraum kann evakuiert bzw. ent-und und belüftet werden, wodurch die Wand und der Druckaufnehmer in kraftschlüssige Verbindung gebracht werden können. Dadurch wird eine präzise Messung von positiven Drücken und negativen Drücken im Bereich des Umgebungsdrucks ermöglicht.

Nachteilig an diesen Ausführungsformen ist, dass der mit Vakuum bzw. Unterdruck beaufschlagte Zwischenraum, dessen Abdichtungen und Zuleitungen schwer sauber zu halten sind. Darüber hinaus können durch das anliegende Vakuum aus dem unsterilen Umfeld Verunreinigungen in den Zwischenraum gesaugt werden, welche die Sterilität des Innenraums des Schlauchbestecks gefährden können.

Aus der DE 10129455 Al ist eine Vorrichtung zum Messen des Druck in einer Fluidleitung bekannt, mit einer Fluidleitung, die einen Abzweig zu einer Fluidkammer mit einer Messmembran aufweist.

Aufgabe der vorliegenden Erfindung ist es, eine Vorrichtung zum Messen des Drucks der eingangs genannten Art, die insbesondere für den Einsatz in der minimal-invasiven Chirurgie bestimmt ist, so zu verbessern, dass einer der erwähnten Nachteile der bekannten Vorrichtungen vermieden wird. Eine präzise Messung des Drucks soll in Bereichen nahe dem Umgebungsdrucks ermöglicht werden. Die Sauberkeit der Vorrichtung und die Sterilität des Fluids soll in geeigneter Weise gewährleistet werden.

Diese Aufgabe wird gemäß der Erfindung bei einer Vorrichtung zum Messen des Drucks der eingangs genannten Art dadurch gelöst, dass die Fluidkammer eine Öffnung zu einem Abzweig aufweist und in den Abzweig der Fluidkammer mindestens ein Ende eines Übertragungselements eingebracht ist und das Übertragungselement eine kraftschlüssige Verbindung zwischen der Fluidkammer und dem Druckaufnehmer ausbildet und das Übertragungselement zur Übertragung des Fluiddrucks auf den Druckaufnehmer geeignet ist.

Durch das Übertragungselement wird der Fluiddruck, der in der Fluidkammer anliegt, aufgenommen und an eine Schnittstelle weitergeleitet. Das Übertragungselement stellt eine mechanisch ausgeformte Signalübertragungsstrecke dar, die den Druck in der Fluidkammer zu der Schnittstelle weiterleitet. Dieser Teil der Signalübertragungsstrecke ist steril ausgeführt. Die Schnittstelle stellt eine kraftschlüssige Verbindung zwischen dem Übertragungselement und dem Druckaufnehmer her, wodurch die Erfasssung des Drucks durch den Druckaufnehmer ermöglicht wird. Die Schnittstelle schirmt den Innenraum der Fluidkammer im Wesentlichen steril ab.

Damit kann auf überraschend einfache Weise die bisher bestehende Notwendigkeit für einen mit Vakuum beaufschlagten Zwischenraum umgangen werden. Die Konstruktion wird dadurch erheblich vereinfacht. Es werden weniger Bauteile benötigt, da beispielsweise die Notwendigkeit für weitere Dichtungsringe, Zu- und Ableitungen entfallen. Darüber hinaus wird beispielsweise eine Pumpe zum Erzeugen des Unterdrucks eingespart. Dies hat eine dramatische Verbilligung der Vorrichtung bei deutlich besserer Reinigbarkeit und einfacherer Handhabung zur Folge.

Die Öffnung zu dem Abzweig der Fluidkammer kann in der Wandung der Fluidkammer längs zur Flussrichtung des Fluids angeordnet sein. In den Abzweig der Fluidkammer wird ein Übertragungselement, insbesondere mindestens ein Ende eines Übertragungselements, eingebracht, das den Abgriff des Drucks ermöglicht, mit dem die Fluidleitung beaufschlagt ist. Es besteht weitestgehend Freiheit in der Wahl des Materials, der Art und des Aufbaus des Übertragungselements, das eine kraftschlüssige Verbindung zwischen der Fluidkammer und dem Druckaufnehmer gewährleistet. Besonders vorteilhafte Ausbildungen der Erfindung sind Gegenstand von Unteransprüchen.

Die kraftschlüssige Verbindung ermöglicht eine im Wesentlichen unverfälschte Übertragung des Drucks von der Fluidleitung über die angeschlossene Fluidkammer und das Übertragungselement zum Druckaufnehmer. Der Druckaufnehmer erfasst, gegebenenfalls nach einer Kalibrierung, den Wert des Drucks in der Fluidleitung.

In einer bevorzugten Ausgestaltung der erfindungsgemäßen Vorrichtung ist die Fluidkammer als Durchflusskammer ausgebildet. Die Durchflusskammer kann Teil eines Kunststoffschlauchs sein, der zu einem eingangs erwähnten Schlauchbesteck gehört, wie es in der Endoskopie verwendet wird.

Diese Maßnahme hat den Vorteil, dass der in der Fluidkammer herrschende Druck im Wesentlichen dem Druck entspricht, mit dem die Fluidleitung beaufschlagt ist. Die Verfälschung der Druckmesswerte durch ungünstige hydrodynamische Strömungsverhältnisse, die leicht in der Umgebung von Senken oder Hohlräumen entstehen können und zur Bildung von Wirbeln und turbulenter Strömung beitragen können, kann effizient reduziert bzw. vermieden werden. Bei einer Fluidkammer, die nur eine Verbindung als Zu- und Ableitung des Fluids aufweist, können beispielsweise auch Verengungen oder Verstopfungen auftreten, die dazu führen, dass der Druck des Fluids in der Fluidkammer nicht dem Druck des Fluids in der Fluidleitung entspricht.

In einer weiteren bevorzugten Ausgestaltung sind das Fluideintrittsende der Fluidkammer, die als Durchlasskammer ausgebildet ist, und das Fluidaustrittsende der Fluidkammer lösbar mit der Fluidleitung verbunden.

Diese Maßnahme hat den Vorteil, dass die Fluidkammer als ein Bauteil oder eine Baugruppe gefertigt werden kann, die austauschbar sind. Dadurch kann die Fluidkammer aus Spritzgussteilen hergestellt werden, die beispielsweise mittels eines Ultraschall-Verschweißverfahrens stoffschlüssig zusammengefügt werden. Durch die Anwendbarkeit dieses Herstellungsverfahrens können hohe Stückzahlen bei geringen Stückkosten erreicht werden

Ein weiterer Vorteil dieser Maßnahme ist darin zu sehen, dass die Fluidkammer als billiges Einwegprodukt steril ausgeführt werden kann, das unter sterilen Bedingungen hergestellt wird und nach Gebrauch verworfen werden kann, wodurch aufwändige und kostspielige Sterilisierungsprozeduren entfallen. So kann die Fluidkammer Bestandteil eines Schlauchbestecks, insbesondere als Verbindungselement (Konnektor) des Schlauchsegments, das in eine Rollenpumpe eingesetzt wird, und den Zu- und Ableitungen des Fluids, die üblicherweise als Kunststoffschläuche ausgebildet sind, vertrieben werden. Dadurch kann in optimaler Weise die Sterilität im Operationssaal aufrecht erhalten werden.

Alternativ kann die Fluidkammer auch in wiederverwendbarer Form hergestellt werden, die dampfsterilisierbar oder autoklavierbar ist. Durch die Lösbarkeit der Fluidleitung ist die Fluidkammer zum Vertrieb als Komponente eines medizinischen Geräts geeignet, beispielsweise einer Rollenpumpe zur Förderung von Spülflüssigkeiten zur Reinigung von Gelenken in der Arthroskopie. Das zugehörige Schlauchbesteck kann mit den entsprechenden Schläuchen zur Befestigung an der Fluidkammer der Rollenpumpe als separates Produkt vertrieben werden,

In einer weiteren besonders bevorzugten Ausgestaltung ist das Übertragungselement mit einer Vorspannung beaufschlagt.

Unter Vorspannung im Sinne der Erfindung wird verstanden, dass der Druckaufnehmer einen im Wesentlichen konstanten Druck erfasst, der vom Betrag größer Null ist, der durch das Übertragungselement erzeugt und zum Druckaufnehmer weitergeleitet wird.

Diese Maßnahme verbessert in vorteilhafter Weise das Erfassen von negativen Drücken und erlaubt insbesondere präzisere Messungen von dynamischen oder statischen Druckbereichen in der Nähe des Umgebungsdrucks und verbessert die lineare Abhängigkeit des erfassten Drucks vom Druck des Fluids in der Fluidkammer.

Eine besonders vorteilhafte Vorspannung liegt bei geeigneten Ausführungsformen im Bereich von 0.5 bar bis 1.5 bar. Bei besonders vorteilhaften Ausführungsformen beträgt die Vorspannung ungefähr 1 bar.

Eine Kalibrierung der Vorrichtung kann beispielsweise bei einer im Wesentlichen ungefüllten oder mit einem Vakuum beaufschlagt Fluidleitung mittels des positiven Drucks durchgeführt werden, der vom Druckaufnehmer erfasst und durch das mit Vorspannung beaufschlagte Übertragungselement erzeugt wird.

Ein weiterer Vorteil dieser Maßnahme ist darin zu sehen, dass der Druckaufnehmer als Überdrucksensor ausgebildet sein kann, die nur zur Erfassung von positiven Drücken geeignet sind und beispielsweise als piezoresistive oder piezoelektrische Drucksensoren kostengünstig im Handel zur Verfügung stehen.

Gehäuse von Druckaufnehmern können eine Füllung aufweisen, wie beispielsweise ein Gel oder eine Silikonfüllung, die von einer Membran umschlossen sein kann. Die Füllung dient der Weiterleitung des Drucks der Membran des Druckaufnehmergehäuses zum Druckaufnehmer. Sie kann das letzte Glied der Druckübertragungskette darstellen, die ausgehend von dem Übertragungselement, das den Fluiddruck abgreift, den Druck auf den Druckaufnehmer überträgt. Die Vorspannung, mit der das Gehäuse des Druckaufnehmers beaufschlagt wird, führt zu einer im Wesentlichen homogenen Druckverteilung in der Füllung, wodurch ein Ausweichen bzw. eine inhomogene Verteilung der Füllung vermieden wird. Dadurch wird in vorteilhafter Weise ein Driften, der durch den Druckaufnehmer erfassten Drücke bzw. Druckmesswerte, die sich in etwa anteilig aus der Vorspannung und dem Fluiddruck summarisch ergeben, vermieden.

Darüber hinaus ermöglicht die Erfassung der Vorspannung durch den Druckaufnehmer die Überprüfung des korrekten Einbaus der Verbindungselemente (Konnektoren) des Schlauchbestecks, die Kontrolle des Befüllungsvorgangs des Schlauchbestecks mit dem Fluid, die Überwachung der Steuerung des medizinischen Geräts, eine anwendungsnahe Kalibrierung des Drucks sowie eine Parametererfassung für die Regelung des medizinischen Geräts, das insbesondere als Rollenpumpe ausgebildet sein kann.

In einer weiteren bevorzugten Ausgestaltung der Erfindung ist die Vorspannung, mit der das Übertragungselement beaufschlagt wird, einstellbar ausgebildet.

Diese Maßnahme hat den Vorteil, dass der Wert des positiven Drucks, der am Druckaufnehmer erfasst und durch das vorgespannte Übertragungselement erzeugt wird, situationsspezifisch der konkreten medizinischen Anwendung angepasst werden kann, was die Sicherheit des Patienten im Rahmen der durchgeführten medizinischen Anwendung erhöht.

In einer weiteren bevorzugten Ausgestaltung der Erfindung ist das Übertragungselement kolbenförmig ausgebildet.

Diese Maßnahme ermöglicht eine besonders einfache und gegebenenfalls automatisierbare Assemblierung der erfindungsgemäßen Vorrichtung, da das Ende eines kolbenförmigen Übertragungselements im Rahmen des Assemblierungsprozesses besonders leicht in die Öffnung der Fluidkammer eingebracht werden kann.

Das kolbenförmige Übertragungselement kann Bohrungen bzw. Perforationen aufweisen. Dadurch kann das Fluid durch das Übertragungselement hindurch strömen, was in besonders vorteilhafter Weise eine homogene Verteilung des Fluids und des Fluiddrucks um das Übertragungselement erzeugt. Dadurch wird die Präzision der Erfassung des Fluiddrucks durch den Druckaufnehmer erhöht, was zur Sicherheit bei der klinischen Anwendung beiträgt.

In einer weiteren bevorzugten Ausgestaltung der Erfindung ist das Übertragungselement mit mindestens einer Federanordnung oder mit mindestens einer Magnetanordnung versehen, die zum Erzeugen der Vorspannung geeignet sind.

Diese Maßnahme hat den Vorteil, dass Federanordnungen oder Magnetanordnungen in unterschiedlichsten Größen und Formen mit unterschiedlichsten Federhärten bzw. magnetischen Kenngrößen herstellbar, kostengünstig im Handel beziehbar und einfach verbaubar sind. Magnetanordnungen sind reibungsfrei, wodurch möglichen Kontaminationen mit Abrieb vorgebeugt wird kann, welche die Sterilität der Vorrichtung gefährden könnten.

Vorzugsweise ist das Übertragungselement einteilig ausgebildet.

Diese Maßnahme hat den Vorteil, dass einteilige Bauteile besonders leicht aus den verschiedensten Materialien gefertigt und an vorgegebene Formen bzw. Geometrien angepasst werden können, da verschiedenste Bearbeitungs- und Herstellungsverfahren wie beispielsweise Walzen, Fräsen, Spritzgießen u.ä. angewendet werden können. Ein einteilig ausgebildetes Bauteil, das einteilig ausgebildet ist, kann besonders einfach sterilisiert werden, da keine Ritzen oder Hohlräume zwischen Einzelteilen auftreten.

In einer alternativen Ausgestaltung der Erfindung ist das Übertragungselement mehrteilig ausgebildet.

Diese Maßnahme hat den Vorteil, dass das Übertragungselement leichter an die zu erfüllenden Funktionen des Abgreifens des Drucks, des Erzeugens und Übertragens einer Vorspannung sowie des Ausbildens einer kraftschlüssigen Verbindung angepasst werden kann. So kann das Übertragungselement beispielsweise verformbare oder bewegliche Bauteile wie eine Feder bzw. einen Kolbenstempel umfassen. Darüber hinaus wird der Austausch defekter Teile ermöglicht, wodurch Wartungsarbeiten flexibler gestaltet werden können.

In einer weiteren bevorzugten Ausgestaltung der Erfindung ist das Übertragungselement austauschbar ausgebildet.

Diese Maßnahme hat den Vorteil, dass bei Defekten des Übertragungselements eine Reparatur der Vorrichtung durch einfachen Austausch des Übertragungselements bewerkstelligt werden kann, ohne die gesamte erfindungsgemäße Vorrichtung ersetzen zu müssen.

In einer weiteren bevorzugten Ausgestaltung der Erfindung ist das dem Druckaufnehmer zugewandte Ende des Übertragungselements von einer Membran umfasst ist, so dass ein fluiddichter Verschluss des Abzweigs der Fluidkammer mit dem Druckaufnehmer zugewandten Ende des Übertragungselements ausgebildet wird.

Diese Maßnahme hat den Vorteil, die Dichtheit der Fluidleitung zu erhöhen bzw. zu gewährleisten und ein Austropfen oder Ausgasen des Fluids zu verhindern.

Darüber hinaus wird der Innenraum der Fluidkammer bzw. der Fluidleitung steril von der weiteren Umgebung abgetrennt, was die Sicherheit erhöht. Dadurch kann auch ein unsteriler Druckaufnehmer verwendet werden, wodurch Kosten eingespart werden und die Herstellung der Vorrichtung vereinfacht wird, die ansonsten aufwändige Sterilisierungsprozeduren des Druckaufnehmers umfassen müsste.

Weiterhin wird die Genauigkeit der Erfassung des Drucks erhöht, da die Membran den Druckaufnehmer gleichmäßig über dessen gesamte Fläche mit Druck beaufschlagt, die mit der Membran in kraftschlüssiger Verbindung steht.

In einer weiteren bevorzugten Ausgestaltung der Erfindung ist der Abzweig der Fluidkammer, in die das Ende des Übertragungselements eingebracht ist, durch einen Dichtungsring oder eine Membran fluiddicht verschlossen ist.

Diese Maßnahme erhöht in besonders einfacher Art und Weise die Dichtheit und Sterilität der Vorrichtung.

In einer weiteren bevorzugten Ausgestaltung der Erfindung ist das in den Abzweig der Fluidkammer eingebrachte Ende des Übertragungselements durch ein Silikonöl gleitend gelagert und bildet mittels eines Dichtelements einen fluiddichten Verschluss der Öffnung aus.

Diese Maßnahme hat den Vorteil, dass durch die gleitende Lagerung des Übertragungselements die Übertragung des Drucks auf den Druckaufnehmer besonders einfach und effizient bewerkstelligt wird, ohne die Dichtheit und Sterilität der Fluidkammer zu beeinträchtigen.

In einer weiteren bevorzugten Ausgestaltung der Erfindung weist die Vorrichtung mehrere Übertragungselemente und mehrere Druckaufnehmer sowie eine Fluidkammer mit mehreren Öffnungen zu mehreren Abzweigen auf.

Diese Maßnahme hat den Vorteil, dass mehrere Druckaufnehmer zur gegenseitigen Überwachung eingesetzt werden können. Dadurch können beispielsweise weitere Regelungskreisläufe aufgebaut werden, die zur Erhöhung der Sicherheit der Vorrichtung eingesetzt werden können.

Weiterhin können die Messverstärker und die Temperaturkompensation für die mehreren Druckaufnehmer in einem Schaltungslayout auf einem Print bzw. einer Platine angeordnet sein. Dadurch können erhebliche Kostenvorteile und Platzersparnisse erzielt werden.

In einer weiteren bevorzugten Ausgestaltung der Erfindung ist mindestens eine Membran vorgesehen ist, die einen fluiddichten Verschluss der mehreren Abzweige der Fluidkammer ausbildet und der Verschluss den Innenraum der Fluidkammer steril hält.

Diese Maßnahme hat den Vorteil, dass die mehreren Abzweige der Fluidkammer durch beispielsweise nur eine einzige Membran oder eine einzige Doppelmembran abgedichtet und steril gehalten werden können. Dadurch wird die Anzahl der Komponenten der Vorrichtung gering gehalten, was die Herstellung verbilligt und vereinfacht. Eine geringe Anzahl an Komponenten trägt zur Reduktion der Ausfallswahrscheinlichkeit der Vorrichtung bei.

In einer weiteren bevorzugten Ausgestaltung der Erfindung weisen die Außenwände der Fluidkammer mindestens eine Vertiefung aus, die als Positionierungshilfe geeignet ist.

Diese Maßnahme hat den Vorteil, dass die mindestens eine Vertiefung der Außenwände der Fluidkammer, die beispielsweise als seitliche Rille oder Rillen ausgebildet sein können, das Platzieren und Verbauen der Fluidkammer vereinfachen.

Dadurch kann die Fluidkammer beispielsweise als Verbindungselement (Konnektor) eines Schlauchbestecks verwendet werden, das durch die passgenaue Vertiefung präziser in einem medizinischen Gerät, insbesondere eine Rollenpumpe, an der vorgesehenen Stelle platziert werden kann. Dadurch kann beispielsweise das Auswechseln des Schlauchbestecks einfacher und schneller erfolgen, wodurch Arbeitszeit und Kosten bei dem medizinischen Bedienpersonal eingespart werden können, das mit den entsprechenden Aufgaben der Wartung und Bedienung des medizinischen Geräts betraut ist. Darüber hinaus kann die Sterilität im Operationsbereich einfacher gewährleistet werden.

In einer weiteren bevorzugten Ausgestaltung der Erfindung ist die Fluidkammer durch einen Hebel im Gehäuse eines medizinischen Gerätes verriegelt, wobei der Hebel die Fluidkammer mit einer Kraft beaufschlagt, die ein Vielfaches der Summe aus Vorspannung und Druck des Fluids entspricht.

Diese Maßnahme hat den Vorteil, dass die Fluidkammer in einer kraftschlüssigen Verbindung mit dem Gehäuse des medizinischen Gerätes steht. Der Druckaufnehmer kann im Gehäuse eines medizinischen Geräts angeordnet sein. Durch den Kraftschluss, den der Hebel zwischen Fluidkammer und dem Gehäuse ausbildet, wird in besonders vorteilhafter Weise eine Erfassung des Fluiddrucks in der Fluidkammer ermöglicht. Diese Maßnahme gestattet den Austausch der Fluidkammer, die beispielsweise als Teil eines Schlauchbestecks lösbar von der Fluidleitung ausgebildet sein kann.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend an einem besonders bevorzugten Ausführungsbeispiel in Zusammenhang mit den beiliegenden Zeichnungen näher beschrieben und erläutert. Es zeigen:
- Fig. 1: eine schematische Darstellung einer erfindungsgemäßen Vorrichtung im Längsschnitt
- Fig. 2: eine Darstellung einer Fluidkammer einer erfindungsgemäßen Druckmessvorrichtung im Querschnitt
- Fig. 3: eine Darstellung einer Fluidkammer mit verbundener Fluidleitung, Übertragungselement und abdichtender Membran einer erfindungsgemäßen Druckmessvorrichtung
- Fig. 4: eine Darstellung einer erfindungsgemäßen Vorrichtung, die zur bestimmungsgemäßen Verwendung in einer Rollenpumpe angeordnet ist.

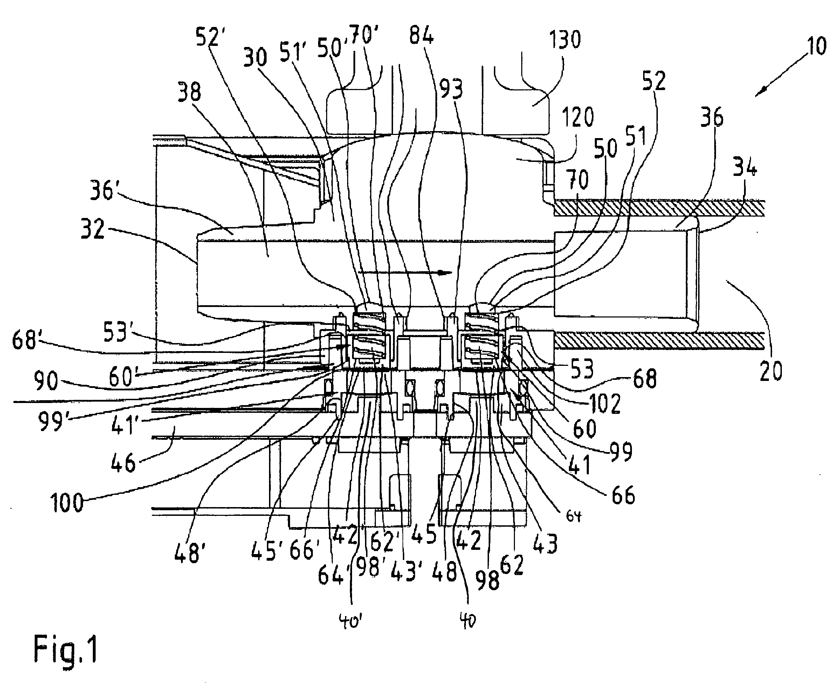

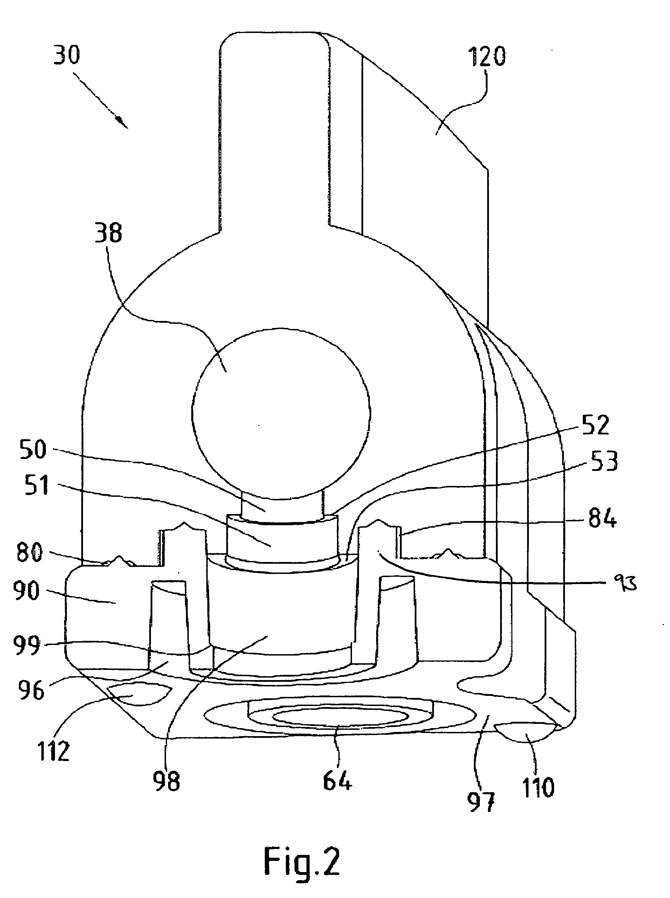

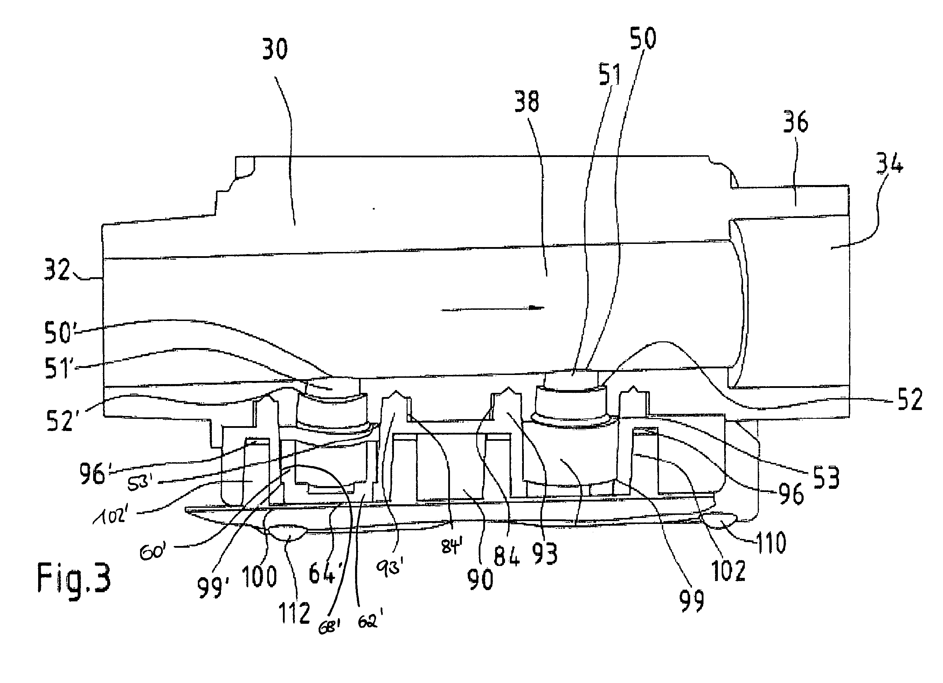

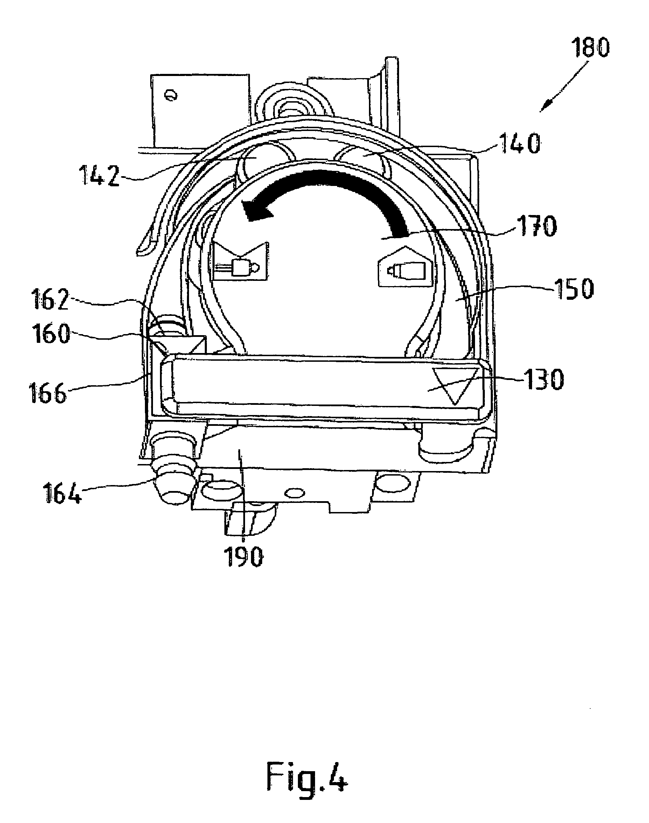

In Fig. 1 ist eine mit dem allgemeinen Bezugszeichen 10 versehene erfindungsgemäße Vorrichtung zum Messen des Drucks in einer Fluidleitung 20 im Längsschnitt dargestellt. Weitere Einzelheiten der Druckmessvorrichtung 10 sind in den Fig. 2 und 3 gezeigt, wobei gleiche, gleichartige oder vergleichbare Bauteile dasselbe Bezugszeichen aufweisen. Gestrichene Bezugszeichen werden zur Bezeichnung von Bauteilen verwendet, die als Doppel in gleicher Ausführung dargestellt sind wie die Bauteile, die mit ungestrichenen Bezugszeichen versehen sind. In Fig. 4 ist eine erfindungsgemäße Vorrichtung in Zusammenhang mit einer Rollenpumpe dargestellt, wie sie üblicherweise im klinischen Alltag zum Einsatz kommt.

Die Druckmessvorrichtung 10 weist eine Fluidkammer 30 auf, die als Durchlasskammer ausgebildet ist. Die Fluidkammer 30 ist am Fluideintrittsende 32 und am Fluidaustrittsende 34 lösbar mit einer Fluidleitung 20 verbunden. Die Fluidleitung 20 ist als Kunststoffschlauch ausgebildet, der über den Anschluss 36 der Fluidkammer 30 geschoben wird und an dem Fluidaustrittsende 34 formschlüssig anliegt.

Der Innenraum 38 der Fluidkammer 30 weist neben dem Fluideintrittsende 32 und dem Fluidaustrittsende 34 zwei weitere Öffnungen 50, 50' zu Abzweigen 51,51' auf, die in der Wandung der Fluidkammer 30 längs zur Flussrichtung des Fluids angeordnet sind, die durch die Richtung des Pfeils in Fig. 1 angezeigt wird.

An den ringförmigen Ansatzflächen 52, 52' in den Abzweigen 51, 51' sind Federn 70, 70' der Übertragungselemente 60, 60' abgestützt. Die Federn 70, 70' liegen an den ringförmigen Ansatzflächen 66, 66' in den kolbenförmigen Elementen 62, 62' der Übertragungselemente 60, 60' an. Die kolbenförmigen Elemente 62, 62' sind zwischen den ringförmigen Anschlagflächen 53, 53' und den ringförmigen Anschlagflächen 99, 99' einer Bodenplatte 90 beweglich angeordnet. Die kolbenförmigen Elemente 62, 62' weisen Vorsprünge 68, 68'. Die Vorsprünge 68, 68' bilden Anschlagflächen aus, die mit den Anschlagflächen 99, 99' der Bodenplatte überlappen, wodurch der Hub der Bewegung der kolbenförmigen Elemente 62, 62' begrenzt wird und die Übertragungselemente 60, 60' im Durchgang 98, 98' der Bodenplatte 90 gehalten werden.

Die Federn 70, 70' der Übertragungselemente 60, 60' sind so gewählt, dass im entspannten Zustand ihre Länge größer ist als der Abstand der Ansatzflächen 52, 52' und der Ansatzflächen 99, 99'.

Die Abzweige 51, 51', die anschließenden Durchgänge 98, 98' der Bodenplatte 90 sind von dem Fluid ausgefüllt. Auch die kolbenförmigen Elemente 62, 62' sind mit dem Fluid befüllt und können vom Fluid umspült sein. Die kolbenförmigen Elemente können Perforationen bzw. Durchbohrungen (nicht dargestellt) aufweisen, die eine homogenere Verteilung des Fluids in den Durchgängen 98, 98' und damit des Fluiddrucks ermöglichen.

Die kolbenförmigen Elemente 62, 62' der Übertragungselemente 60, 60' sind mit dem Druck des Fluids und der Vorspannung der Federn 70, 70' beaufschlagt. Die Enden 64, 64' der Übertragungselemente 60, 60' wirken auf eine Membran 100 ein, die sie mit einem Druck beaufschlagen, der im Wesentlichen anteilig die Vorspannung der Federn 70, 70' und den Fluiddruck umfasst.

Die Membran 100 umfasst die Enden 64, 64' der Übertragungselemente 60, 60' und bildet einen flüssigkeitsdichten Verschluss, wodurch ein Austreten bzw. Aussickern des Fluids aus der Fluidleitung 20, dem Innenraum 38 der Fluidkammer 30, den Abzweigen 51, 51' und den Durchgängen 98, 98' verhindert wird.

In Fig. 2 ist der Aufbau der Fluidkammer 30 im Querschnitt gezeigt. Der Innenraum der Fluidkammer weist eine Öffnung 50 zu einem Abzweig 51 auf. Der Abzweig weist eine ringförmige Absatzfläche 52 auf. Die kreisförmige Ansatzfläche 52 dient als Abstützfläche für eine Feder 70 (nicht dargestellt).

Die Fluidkammer 30 weist ringförmige Ausnehmungen 84, 84' auf. Nach Einsetzen der Feder an die Ansatzfläche 52 des Abzweigs 51 und des kolbenförmigen Elements 62 an die Ansatzfläche 53 des Abzweigs 51 wird der axiale Bewegungsbereich des Übertragungselements 60 in dem Durchgang 98 einer Bodenplatte 90 festgelegt, da der Durchgang 98 eine ringförmige Ansatzfläche 99 aufweist.

Die Bodenplatte 90 weist ringförmige Vorsprünge 93, 93' auf, die sich in Ausnehmungen 84, 84' der Fluidkammer 30 einfügen. Durch Ultraschallschweißen kann eine stoffschlüssige Verbindung zwischen der Fluidkammer 30 und der Bodenplatte 90, die jeweils aus Kunststoffen wie Polyethylen oder Polypropylen gefertigt sind, hergestellt werden. Dadurch wird das Übertragungselement 60 im Durchgang 98 der Bodenplatte 90 zwischen der Ansatzfläche 52 und der Ansatzfläche 99 eingefangen.

Das dem Druckaufnehmer 40 zugewandte Ende 64 des Übertragungselements 60 bildet einen in etwa bündigen Abschluss mit der Unterseite 97 der Bodenplatte 90.

Die Bodenplatte 90 weist an ihrer Unterseite 97 kreisförmige Ausnehmungen 96, 96' auf. Aus Fig. 3 ist ersichtlich, dass die Ausnehmungen 96, 96' in etwa konzentrisch um die Durchgänge 98, 98' der Bodenplatte 90 angeordnet sind.

Die Membran 100, die beispielsweise aus Silikon gefertigt und steril ist, weist kreisförmige Vorsprünge 102, 102' auf, die sich formschlüssig in die Ausnehmungen 96, 96' der Bodenplatte 90 einpassen. Die Membran 100 kann dadurch besonders einfach positioniert und mit der Bodenplatte zusammengefügt werden.

Die Membran 100 bildet einen fluiddichten Verschluss der Bodenplatte 90 und weist eine in etwa ebene Außenfläche auf, die mit den Membranen 42, 42' der Gehäuse 41, 41' der Druckaufnehmer 40, 40' in kraftschlüssiger Verbindung steht (siehe auch Fig. 1).

Die Wände 46 und 48' der Gehäuse 41, 41' sind in Ausnehmungen 45, 45' der Platte 46 eingepasst. Die Druckaufnehmer 40, 40' sind auf der Platte 46 angeordnet, die als Platine mit aufgedruckten Schaltkreisen ausgebildet sein kann. Die Druckaufnehmer 40, 40' können beispielsweise als Schaltkreise auf der Platte 46 aufgedruckt oder eingeätzt sein.

Die Innenräume der Gehäuse 41, 41' sind mit Silikon 43 ausgefüllt, das zur Weiterleitung des Drucks von den Membranen 41, 41' zu den Druckaufnehmern 40, 40' dient. Die Vorspannung, mit der die Membranen 41, 41' beaufschlagt werden, verhindert ein Ausweichen der Silikonfüllung 43 in den Gehäusen 41, 41', das eine Drift in der Erfassung des Drucks durch die Druckaufnehmer 40, 40' bewirken könnte.

Die Bodenplatte weist Füße 110, 112, 114 auf, die halbkugelförmig ausgebildet sind. In Fig. 4 ist ein Verbindungselement (Konnektor) 160 gezeigt, das aus einer ultraschallverschweißten Fluidkammer 30 mit Bodenplatte 90 besteht. Nach Einfügen der Membran 100 in die Bodenplatte 90 dienen die Füße 110, 112, 114 zur Positionierung des Verbindungselements 160 im Gehäuse 190 einer Rollenpumpe 180. Dazu weist das Gehäuse 190 entsprechende Mulden (nicht dargestellt) im Schlauchbett 150 auf, die der Aufnahme der halbkugelförmigen Füße 110, 112, 114 dienen.

An der Außenwand 166 weist das Verbindungselement 160 eine Rille (nicht dargestellt) auf, die das genaue Einsetzen des Verbindungselements 160 in das Schlauchbett 150 des Gehäuses 190 der Rollenpumpe 180 vereinfacht.

Durch einen Drehhebel 130 wird das Verbindungselement 160 im Schlauchbett 150 der Rollenpumpe 180 verriegelt. Der Hebel 130 drückt auf den Kamm 120 der Fluidkammer 30 und bewirkt einen Kraftschluss zwischen der Bodenplatte 90 und dem Gehäuse 190 der Rollenpumpe 180. Der Hebel 130 übt eine Kraft auf die Fluidkammer 30 aus, die ein Vielfaches der Summe aus Vorspannung und Druck des Fluids entspricht.

Das Verbindungselement 160 weist Anschlüsse 162 und 164 auf. Der Anschluss 162 dient zum Anschließen eines Schlauchsegments aus vorzugsweise beständigerem Kunststoff (nicht dargestellt) an das Verbindungselement 160. Durch die Rollen 140 und 142 (und vier weitere nicht dargestellte Rollen), die auf einem Rollenkopf 170 angeordnet sind, wird das nicht dargestellte Schlauchsegment eingedrückt bzw. abgequetscht, wodurch bei Rotation des Rollenkopfes 120 eine peristaltische Wirkung erzielt wird, die zur Förderung des Fluids verwendet wird.

Der Anschluss 164 dient zum Anschließen eines weiteren Kunststoffschlauchs eines Schlauchbestecks, der als Zuleitung des Fluids dient.

## Patentansprüche

1. Vorrichtung (10) zum Messen des Drucks in einer Fluidleitung (20) mit einer Fluidkammer (30), die fluidleitend mit der Fluidleitung verbunden ist, und mit einem Druckaufnehmer (40, 40'), wobei die Fluidkammer (30) eine Öffnung zu einem Abzweig (51, 51') aufweist, **dadurch gekennzeichnet, dass** in den Abzweig, (51, 51') der Fluidkammer (30) mindestens ein Ende eines Übertragungselements (60, 60') eingebracht ist und das Übertragungselement (60, 60') eine kraftschlüssige Verbindung zwischen der Fluidkammer (30) und dem Druckaufnehmer (40, 40') ausbildet und das Übertragungselement (60, 60') zur Übertragung des Fluiddrucks auf den Druckaufnehmer (40, 40') geeignet ist.

2. Vorrichtung nach Anspruch 1, **dadurch gekennzeichnet, dass** die Fluidkammer (30) als Durchflusskammer ausgebildet ist.

3. Vorrichtung nach Anspruch 2, **dadurch gekennzeichnet, dass** das Fluideintrittsende (32) der Durchflusskammer (30) und das Fluidaustrittsende (34) der Durchflusskammer (30) mit der Fluidleitung (20) lösbar verbunden sind.

4. Vorrichtung nach einem der Ansprüche 1 bis 3, **dadurch gekennzeichnet, dass** das Übertragungselement (60, 60') mit einer Vorspannung beaufschlagt ist.

5. Vorrichtung nach Anspruch 4, **dadurch gekennzeichnet, dass** die Vorspannung, mit der das Übertragungselement (60, 60') beaufschlagt wird, einstellbar ausgebildet ist.

6. Vorrichtung nach einem der Ansprüche 1 bis 5, **dadurch gekennzeichnet, dass** das Übertragungselement (60, 60') kolbenförmig ist.

7. Vorrichtung nach Anspruch 1 bis 6, **dadurch gekennzeichnet, dass** das Übertragungselement (60, 60') mit mindestens einer Federanordnung (70, 70') oder mit mindestens einer Magnetanordnung versehen ist, die zum Erzeugen einer Vorspannung geeignet ist.

8. Vorrichtung nach Anspruch 1 bis 7, **dadurch gekennzeichnet, dass** das Übertragungselement (60, 60') einteilig ausgebildet ist.

9. Vorrichtung nach Anspruch 1 bis 7, **dadurch gekennzeichnet, dass** das Übertragungselement (60, 60') mehrteilig ausgebildet ist.

10. Vorrichtung nach Anspruch 1 bis 9, **dadurch gekennzeichnet, dass** das Übertragungselement (60, 60') austauschbar ausgebildet ist.

11. Vorrichtung nach einem der Ansprüche 1 bis 10, **dadurch gekennzeichnet, dass** das dem Druckaufnehmer (40, 40') zugewandte Ende (64, 64') des Übertragungselements (60, 60') von einer Membran (42, 42') umfasst ist, so dass ein fluiddichter Verschluss des Abzweigs (51, 51') der Fluidkammer (30) mit dem dem Druckaufnehmer (40, 40') zugewandtenEnde (64, 64') des Übertragungselements (60, 60') ausgebildet wird.

12. Vorrichtung nach einem der Ansprüche 1 bis 11, **dadurch gekennzeichnet, dass** der Abzweig (51, 51') der Fluidkammer (30), in den das mindestens eine Ende des Übertragungselements (60, 60') eingebracht ist, durch einen Dichtungsring oder eine Membran fluiddicht verschlossen ist.

13. Vorrichtung nach einem der Ansprüche 1 bis 12, **dadurch gekennzeichnet, dass** das in den Abzweig (51, 51') der Fluidkammer (30) eingebrachte Ende des Übertragungselements (60, 60') durch ein Silikonöl gleitend gelagert ist und mittels eines Dichtelements einen fluiddichten Verschluss des Abzweigs ausbildet.

14. Vorrichtung nach einem der Ansprüche 1 bis 13, **dadurch gekennzeichnet, dass** die Vorrichtung (10) mehrere Übertragungselemente (60, 60') und mehrere Druckaufnehmer (40, 40') und eine Fluidkammer (30) mit mehreren Öffnungen (50, 50') zu mehreren Abzweigen (51, 51') aufweist.

15. Vorrichtung nach Anspruch 14, **dadurch gekennzeichnet, dass** mindestens eine Membran (100) vorgesehen ist, die einen fluiddichten Verschluss der mehreren Abzweige (51, 51') der Fluidkammer (30) ausbildet und der Verschluss den Innenraum (38) der Fluidkammer (30) steril hält.

16. Vorrichtung nach einem der Ansprüche 1 bis 15, **dadurch gekennzeichnet, dass** die Außenwände der Fluidkammer (30) mindestens eine Vertiefung, die als Positionierungshilfe geeignet ist, aufweisen.

## Claims

1. Device (10) for measuring the pressure in a fluid line (20), having a fluid chamber (30) which is connected in a fluid-carrying manner to the fluid line, and having a pressure transducer (40, 40'), wherein the fluid chamber (30) has an opening to a branch (51, 51'), **characterized in that** at least one end of a transmission element (60, 60') is introduced into the branch (51, 51') of the fluid chamber (30) and the transmission element (60, 60') forms a non-positively locking connection between the fluid chamber (30) and the pressure transducer (40, 40') and the transmission element (60, 60') is suitable for transmitting the fluid pressure to the pressure transducer (40, 40').

2. Device according to Claim 1, **characterized in that** the fluid chamber (30) is in the form of a flow chamber.

3. Device according to Claim 2, **characterized in that** the fluid inlet end (32) of the flow chamber (30) and the fluid outlet end (34) of the flow chamber (30) are connected detachably to the fluid line (20).

4. Device according to one of Claims 1 to 3, **characterized in that** the transmission element (60, 60') is preloaded.

5. Device according to Claim 4, **characterized in that** the preload applied to the transmission element (60, 60') is designed to be adjustable.

6. Device according to one of Claims 1 to 5, **characterized in that** the transmission element (60, 60') is in the form of a piston.

7. Device according to Claims 1 to 6, **characterized in that** the transmission element (60, 60') is provided with at least one spring arrangement (70, 70') or with at least one magnet arrangement which is suitable for generating a preload.

8. Device according to Claims 1 to 7, **characterized in that** the transmission element (60, 60') is designed in one part.

9. Device according to Claims 1 to 7, **characterized in that** the transmission element (60, 60') is designed in multiple parts.

10. Device according to Claims 1 to 9, **characterized in that** the transmission element (60, 60') is designed to be replaceable.

11. Device according to one of Claims 1 to 10, **characterized in that** that end (64, 64') of the transmission element (60, 60') that faces the pressure transducer (40, 40') is covered by a membrane (42, 42') so that a fluid-tight closure of the branch (51, 51') of the fluid chamber (30) is formed with that end (64, 64') of the transmission element (60, 60') that faces the pressure transducer (40, 40').

12. Device according to one of Claims 1 to 11, **characterized in that** the branch (51, 51') of the fluid chamber (30), into which branch (51, 51') the at least one end of the transmission element (60, 60') is introduced, is closed in a fluid-tight manner by a sealing ring or a membrane.

13. Device according to one of Claims 1 to 12, **characterized in that** that end of the transmission element (60, 60') that is introduced into the branch (51, 51') of the fluid chamber (30) is slidingly mounted by way of a silicone oil and forms a fluid-tight closure of the branch by means of a sealing element.

14. Device according to one of Claims 1 to 13, **characterized in that** the device (10) has a plurality of transmission elements (60, 60') and a plurality of pressure transducers (40, 40') and a fluid chamber (30) having a plurality of openings (50, 50') to a plurality of branches (51, 51').

15. Device according to Claim 14, **characterized in that** there is provided at least one membrane (100) which forms a fluid-tight closure of the plurality of branches (51, 51') of the fluid chamber (30) and the closure keeps the interior (38) of the fluid chamber (30) sterile.

16. Device according to one of Claims 1 to 15, **characterized in that** the outside walls of the fluid chamber (30) have at least one depression which is suitable as a positioning aid.

## Revendications

1. Dispositif (10) de mesure de la pression dans une conduite de fluide (20) comprenant une chambre de fluide (30) qui est connectée fluidiquement à la conduite de fluide, et comprenant un capteur de pression (40, 40'), la chambre de fluide (30) présentant une ouverture vers un embranchement (51, 51'), **caractérisé en ce qu'**au moins une extrémité d'un élément de transfert (60, 60') est introduite dans l'embranchement (51, 51') de la chambre de fluide (30), et l'élément de transfert (60, 60') réalise une liaison par engagement par force entre la chambre de fluide (30) et le capteur de pression (40, 40') et l'élément de transfert (60, 60') est prévu pour transférer la pression de fluide au capteur de pression (40, 40').

2. Dispositif selon la revendication 1, **caractérisé en ce que** la chambre de fluide (30) est réalisée sous forme de chambre de débit.

3. Dispositif selon la revendication 2, **caractérisé en ce que** l'extrémité d'entrée de fluide (32) de la chambre de débit (30) et l'extrémité de sortie de fluide (34) de la chambre de débit (30) sont connectées de manière amovible à la conduite de fluide (20).

4. Dispositif selon l'une quelconque des revendications 1 à 3, **caractérisé en ce que** l'élément de transfert (60, 60') est sollicité avec une précontrainte.

5. Dispositif selon la revendication 4, **caractérisé en ce que** la précontrainte avec laquelle l'élément de transfert (60, 60') est sollicité est réalisée de manière ajustable.

6. Dispositif selon l'une quelconque des revendications 1 à 5, **caractérisé en ce que** l'élément de transfert (60, 60') est en forme de piston.

7. Dispositif selon les revendications 1 à 6, **caractérisé en ce que** l'élément de transfert (60, 60') est pourvu d'au moins un agencement de ressort (70, 70') ou d'au moins un agencement magnétique, qui est prévu pour produire une précontrainte.

8. Dispositif selon les revendications 1 à 7, **caractérisé en ce que** l'élément de transfert (60, 60') est réalisé d'une seule pièce.

9. Dispositif selon les revendications 1 à 7, **caractérisé en ce que** l'élément de transfert (60, 60') est réalisé en plusieurs parties.

10. Dispositif selon les revendications 1 à 9, **caractérisé en ce que** l'élément de transfert (60, 60') est réalisé de manière remplaçable.

11. Dispositif selon l'une quelconque des revendications 1 à 10, **caractérisé en ce que** l'extrémité (64, 64') tournée vers le capteur de pression (40, 40') de l'élément de transfert (60, 60') est entourée par une membrane (42, 42') de telle sorte qu'une fermeture étanche aux fluides de l'embranchement (51, 51') de la chambre de fluide (30) soit réalisée avec l'extrémité (64, 64') de l'élément de transfert (60, 60') tournée vers le capteur de pression (40, 40').

12. Dispositif selon l'une quelconque des revendications 1 à 11, **caractérisé en ce que** l'embranchement (51, 51') de la chambre de fluide (30) dans lequel est introduite l'au moins une extrémité de l'élément de transfert (60, 60'), est fermé de manière étanche aux fluides par une bague d'étanchéité ou une membrane.

13. Dispositif selon l'une quelconque des revendications 1 à 12, **caractérisé en ce que** l'extrémité de l'élément de transfert (60, 60') introduite dans l'embranchement (51, 51') de la chambre de fluide (30) est supportée de manière glissante par une huile de silicone, et constitue au moyen d'un élément d'étanchéité une fermeture étanche aux fluides de l'embranchement.

14. Dispositif selon l'une quelconque des revendications 1 à 13, **caractérisé en ce que** le dispositif (10) présente plusieurs éléments de transfert (60, 60') et plusieurs capteurs de pression (40, 40') et une chambre de fluide (30) avec plusieurs ouvertures (50, 50') vers plusieurs embranchements (51, 51').

15. Dispositif selon la revendication 14, **caractérisé en ce qu'**il est prévu au moins une membrane (100) qui constitue une fermeture étanche aux fluides des plusieurs embranchements (51, 51') de la chambre de fluide (30), et la fermeture maintient stérile l'espace interne (38) de la chambre de fluide (30).

16. Dispositif selon l'une quelconque des revendications 1 à 15, **caractérisé en ce que** les parois extérieures de la chambre de fluide (30) présentent au moins un renfoncement qui est prévu en tant qu'aide au positionnement.
